# DEMANDE DE BREVET EUROPEEN

(11) **EP 3 521 816 A1**
(43) Date de publication de la demande: **07.08.2019**
(21) Numéro de dépôt: 18305119.2
(22) Date de dépôt: 06.02.2018
(51) Int. Cl.: G01N 25/18, G01N 33/00

(54) **MÉTHODE DE CONTRÔLE SUR SITE DE LA QUALITÉ DES GAZ LIVRÉS SUR UN SITE INDUSTRIEL CONSOMMATEUR UTILISANT LA TECHNIQUE DE LA CONDUCTIVITÉ THERMIQUE**

(71) Demandeur: L'AIR LIQUIDE, SOCIETE ANONYME POUR L'ETUDE ET L'EXPLOITATION DES PROCEDES GEORGES CLAUDE, 75007 Paris (FR); Carbagas, 3073 Gümligen (CH)
(72) Inventeur: PEYER, Heinz, CH-3073 Gümligen (CH)
(74) Mandataire: Mellul-Bendelac, Sylvie Lisette

(57) **Abrégé**

L'invention propose une méthode de contrôle sur site de la qualité des gaz livrés sur un site consommateur, contrôle effectué lors de la livraison, pour déclarer conforme ou non à un cahier des charges la livraison, se caractérisant en ce que l'on effectue, lors de la livraison, un contrôle du ou des gaz livré(s) par la technique de la conductivité thermique.

## Description

La présente invention concerne le domaine du contrôle de la qualité (de l' « identité ») des gaz industriels livrés sur un site industriel consommateur, tout particulièrement dans l'industrie alimentaire et pharmaceutique.

Ces industries sont notamment concernées par les gaz suivants : l'azote, l'oxygène, le CO₂, ou encore l'argon et le protoxyde d'azote, seuls ou en mélanges.

Il apparaît en effet que ces industries mettent en oeuvre de plus en plus d'exigences réglementaires, où l'acceptabilité d'une livraison de gaz sur le seul certificat d'analyse fourni par le gazier ne suffit plus (certificat qui tenait compte de tests réalisés en amont dans les propres laboratoires du gazier). Ces industries exigent maintenant la réalisation de tests de conformité (quel gaz ? quelle teneur d'un gaz dans un mélange ? etc...) sur leur site industriel, lors de la livraison (à réception).

Ces tests réalisés sur le site industriel considéré, lors de la livraison, nécessitent dès lors un temps important, obligeant le camion de livraison à attendre le résultat des tests, souvent plusieurs heures (à l'aide des méthodes classiques mentionnées dans les pharmacopées), avant d'achever sa livraison et de se diriger vers son client suivant. Ceci représente incontestablement une perte de temps très préjudiciable pour le gazier qui livre, mais aussi un cout, et une perte de temps, et de main d'oeuvre, pour l'entreprise cliente.

Un des objectifs de la présente invention est alors de proposer une nouvelle méthode de contrôle sur site de la qualité (on peut aussi dire de l' « identité ») des gaz livrés sur un site industriel consommateur.

Comme on le verra plus en détails dans ce qui suit, l'invention propose de réaliser sur le site de l'industriel livré, lors de la livraison, un contrôle du gaz ou mélange livré, par la technique de la conductivité thermique.

Selon un des modes de mise en oeuvre de l'invention, on dispose lors de la livraison, d'un dispositif d'identification du gaz lors de sa livraison et de sa réception sur le site consommateur, qui comprend :
- un analyseur de la conductivité thermique des gaz, analyseur qui aura été au préalable calibré à l'aide de chacun des gaz purs susceptibles d'être ultérieurement livrés, seuls ou en mélanges, durant une telle livraison ("calibration") ; et
- un système d'acquisition et de traitement de données,
dispositif d'identification du gaz qui est apte :
- à acquérir un ou des échantillons du gaz ou mélange livré ;
- à acquérir un ou des échantillons d'un gaz de référence tel l'azote, gaz de référence préféré puisqu'il est couramment présent sur de tels sites ;
- à effectuer une ou plusieurs mesures de la conductivité du ou des échantillons du gaz de référence et à effectuer la comparaison des mesures obtenues avec les valeurs de conductivité thermique obtenues en amont lors de ladite « calibration" avec ce même gaz de référence ;
- à effectuer une ou plusieurs mesures de la conductivité du ou des échantillons du gaz ou mélange livré, et à effectuer la comparaison des mesures ainsi obtenues avec les valeurs de conductivité thermique obtenues en amont lors de ladite "calibration" , et
- à donner un diagnostic de type « livraison conforme » ou « livraison non conforme » autorisant la livraison ou non du gaz ou mélange livré.

Et l'on comprend dès lors les avantages qui sont liés à cette proposition technique par rapport aux méthodes actuelles où des échantillons sont envoyés au laboratoire du site livré, où il faut attendre le retour de ce laboratoire etc... :
- cette qualification sur site lors de la livraison se fait en quelques minutes (couramment en moins de 5 minutes) ;
- chaque molécule est caractérisée par une valeur unique de conductivité thermique à une température donnée, et la littérature disponible montre bien que les conductivités thermiques de gaz tels que l'air, N₂, O₂, Ar, CO₂, N₂O, NH₃, C₂H₆, ou encore C₃H₈ sont clairement différentiables, et permettent donc d'attester que tel gaz et non tel autre est incontestablement présent dans l'échantillon testé ;

- nulle nécessité ici de disposer de gaz de calibration pour chacun des gaz ou mélanges livrés : la seule présence sur site (et elle est avérée) d'un gaz de référence tel l'azote, permet de mesurer la conductivité thermique de l'azote à titre de référence, pour confirmer le fait que l'appareillage est bien opérationnel et fournit une valeur conforme à la valeur de la conductivité de l'azote dans les gammes de température pratiquées, et que donc l'analyse de la conductivité thermique du ou des gaz d'intérêt livré(s) peut commencer ;
- aucune interférence entre gaz ne peut perturber le résultat de la qualification ;
- cette méthode est très simple, automatique, et ne nécessite aucune qualification particulière et l'intervention d'aucun laboratoire spécialisé;
- le coût de ce contrôle est bien réduit par rapport aux méthodes actuellement pratiquées (à titre d'exemple, un seul analyseur au lieu de cinq analyseurs pour le contrôle de la livraison de N₂, Ar, O₂, CO₂ et du protoxyde d'azote) ;
- selon un des modes avantageux de mise en oeuvre de l'invention, le dispositif en question, préférentiellement "waterproof" i.e de construction imperméable, est positionné « à demeure » dans la zone de livraison des fluides gaziers du site industriel considéré.

La présente invention concerne alors une méthode de contrôle sur site de la qualité des gaz livrés sur un site industriel consommateur, contrôle effectué lors de la livraison, pour déclarer conforme ou non à un cahier des charges la livraison, se caractérisant en ce que l'on effectue, lors de la livraison, un contrôle du ou des gaz livré(s) par la technique de la conductivité thermique, de la façon suivante :
- on dispose lors de la livraison, d'un dispositif d'identification du gaz lors de sa livraison et de sa réception sur le site consommateur, qui comprend :
   - un analyseur de la conductivité thermique des gaz, analyseur qui aura été au préalable calibré à l'aide de chacun des gaz purs susceptibles d'être ultérieurement livrés, seuls ou en mélanges, durant une telle livraison ("calibration") ; et

   - un système d'acquisition et de traitement de données, dispositif d'identification du gaz qui est apte :
      - à acquérir un ou des échantillons du gaz ou mélange livré ;
      - à acquérir un ou des échantillons d'un gaz de référence tel l'azote, gaz de référence préféré puisqu'il est couramment présent sur de tels sites ;
      - à effectuer une ou plusieurs mesures de la conductivité du ou des échantillons du gaz de référence et à effectuer la comparaison des mesures obtenues avec les valeurs de conductivité thermique obtenues en amont lors de ladite « calibration" avec ce même gaz de référence ;
      - à effectuer une ou plusieurs mesures de la conductivité du ou des échantillons du gaz ou mélange livré, et à effectuer la comparaison des mesures ainsi obtenues avec les valeurs de conductivité thermique obtenues en amont lors de ladite "calibration" , et
      - à donner un diagnostic de type « livraison conforme » ou « livraison non conforme » autorisant la livraison ou non du gaz ou mélange livré.

## Revendications

1. Méthode de contrôle sur site de la qualité des gaz livrés sur un site consommateur, contrôle effectué lors de la livraison, pour déclarer conforme ou non à un cahier des charges la livraison, **se caractérisant en ce que** l'on effectue, lors de la livraison, un contrôle du ou des gaz livré(s) par la technique de la conductivité thermique, de la façon suivante :
- on dispose lors de la livraison, d'un dispositif d'identification du gaz lors de sa livraison et de sa réception sur le site consommateur, qui comprend :
- un analyseur de la conductivité thermique des gaz, analyseur qui aura été au préalable calibré à l'aide de chacun des gaz purs susceptibles d'être ultérieurement livrés, seuls ou en mélanges, durant une telle livraison ("calibration") ; et
- un système d'acquisition et de traitement de données, dispositif d'identification du gaz qui est apte :
- à acquérir un ou des échantillons du gaz ou mélange livré ;
- à acquérir un ou des échantillons d'un gaz de référence tel l'azote, gaz de référence préféré puisqu'il est couramment présent sur de tels sites ;
- à effectuer une ou plusieurs mesures de la conductivité du ou des échantillons du gaz de référence et à effectuer la comparaison des mesures obtenues avec les valeurs de conductivité thermique obtenues en amont lors de ladite « calibration" avec ce même gaz de référence ;
- à effectuer une ou plusieurs mesures de la conductivité du ou des échantillons du gaz ou mélange livré, et à effectuer la comparaison des mesures ainsi obtenues avec les valeurs de conductivité thermique obtenues en amont lors de ladite "calibration" , et
- à donner un diagnostic de type « livraison conforme » ou « livraison non conforme » autorisant la livraison ou non du gaz ou mélange livré.
